# EUROPEAN PATENT APPLICATION

(11) **EP 2 037 237 A2**
(43) Date of publication of application: **18.03.2009**
(21) Application number: 08163127.7
(22) Date of filing: 28.08.2008
(51) Int. Cl.: G01G 19/50, G01G 23/16, A61B 5/053

(54) **Weight Measuring Apparatus**

(30) Priority: 13.09.2007 JP 2007237518
(71) Applicant: TANITA CORPORATION, Tokyo (JP)
(72) Inventor: Inoue, Koki, Itabashi-ku Tokyo (JP)
(74) Representative: Haley, Stephen

(57) **Abstract**

The invention provides a weight measuring apparatus which carries out zero-point reset as an output value of a load signal in an unloaded state when the weight measuring apparatus is not in use, including a weight measuring unit that measures a load applied to a machine body and outputs the load signals; and a control device that receives the load signals from the weight measuring unit at reset time intervals for carrying out the zero-point reset of the weight measuring unit in the unloaded state, in which the control device changes the reset time interval to next zero-point reset according to the difference between the output value of the load signal at the time of previous zero-point reset and the output value of the load signal at zero-point reset of this time.

## Description

### 1.

The present invention relates to a weight measuring apparatus for measuring weight of an object to be measured and, more specifically, to a weight measuring apparatus which carries out zero-point reset when the weight measuring apparatus is not in use.

### 2.

In the related art, various types of weight measuring apparatus for measuring the weight of the object to be measured have been proposed and, specifically, a weighting machine for measuring the weight of a measured person is used. The weighting machine as the weight measuring apparatus generally includes a weight measuring unit in a machine body, and the weight measuring unit includes a load cell composed of a distortable member made up of a metallic member which is deformed according to an applied load and a strain gauge (sensor) bonded to the distortable member. When the distortable member is distorted by a load applied when the measured person rides on an upper surface of the machine body and the strain gauge expands or contracts correspondingly, the weight of the measured person is calculated by using a change in value of resistance (output value) according to the expansion and contraction of the strain gauge as a change in load signal output.

When the weighting machine as described above is used, it is necessary to place the weighting machine horizontally and set the output value of the load cell in an unloaded state, in which the measured person does not ride on the machine body, to zero point, which is called "zero-point reset2. It is because the weight of the measured person is measured on the basis of the difference between an output value of the load cell when the measured person rides on the weighting machine and the output value of the load cell in the unloaded state.

In the case of a multifunctional weighing machine having a function measure biometric data of the measured person other than the weight (for example, a body-fat-meter-integrated weighting machine, a body-composition meter-integrated weighting machine), electrode members for measuring bioimpedance of the measured person are generally arranged on the upper surface of the machine body in addition to the weight measuring unit. By using the bioimpedance measured by flowing a weak constant current from the electrode members to the bottom of the measured person's feet, various biometric data such as the percent body fat are calculated and obtained as an estimated value. However, since the biometric data is calculated with the weight of the measured person as a parameter in most cases, the zero-point reset with high degree of accuracy is important for obtaining accurate biometric data (JP-A-2001-104273).

The weight measuring apparatus in the related art as described above is generally adapted to carry out the zero-point reset after having pushed a measurement start switch (start button) and hence the measurement cannot be started until the zero-point reset is completed. Therefore it is not convenient for the measured person because he/she cannot ride on the machine body and start measurement quickly when he/she feels like measuring the weight or other biometric data. Therefore there is proposed a so-called step-on type weight measuring apparatus which completes the zero-point reset at a predetermined interval when the weighting machine is not in use, and starts up measurement when it detects that the measured person rides on the weighting machine, so that the measurement of the biometric data may be started quickly as soon as the measured person feels like measuring the biometric data.

When the weight measuring apparatus is not in use, the posture of the weight measuring apparatus is changed variously such as being propped against a wall, or being inverted when a battery as a power source is replaced. Therefore, the weight measuring apparatus is not always in a stably installed state. Also, the environment in which the weight measuring apparatus is used varies. For example, the peripheral temperature may change, or the weight measuring apparatus by itself may be subjected to the change with time. Since the zero point varies depending on the change in the state of installation or the change in environment of the weight measuring apparatus, the measurement result of the acquired weight or other biometric data may be different from the actual value. In order to prevent such an event, it is preferable to reset the zero point as the output value of the load cell in the unloaded state to temporal latest data obtained immediately before measurement, so that the change in the state of installation or the environment or the change with time is reflected.

Therefore, in the step-on type weight measuring apparatus, it is desirable to set a reset time interval for the zero-point reset to be carried out when the weighting machine is not in use to a short interval from the view point of obtaining the latest zero point immediately before the measurement. However, it is not preferable to carry out the zero-point reset always in short intervals also when phenomena such as the change in the state of installation or the environment or the change with time of the weighting machine are not apparent because the waste of consumption power is increased.

Therefore, it is an object of the present invention to provide a weight measuring apparatus in which zero-point reset is carried out according to the change in state of installation or environment and the change with time, and reduction of power consumption is achieved.

In order to solve the above-described problems, the invention provides a weight measuring apparatus which carries out zero-point reset as an output value of a load signal in an unloaded state when the weight measuring apparatus is not in use, including a weight measuring unit that measures a load applied to a machine body and outputs the load signals; and a control device that receives the load signals from the weight measuring unit at reset time intervals for carrying out the zero-point reset of the weight measuring unit in the unloaded state, in which the control device changes the reset time interval to next zero-point reset according to the difference between the output value of the load signal at the time of previous zero-point reset and the output value of the load signal at zero-point reset of this time.

Preferably, the control device elongates the reset time intervals when the difference of the output values is smaller than a predetermined value, and shortens the reset time interval when the difference of the output values is equal to or larger than the predetermined value.

Preferably, the control device detects the difference between the output value of the load signal at the previous zero-point reset and the output value of the load signal at the zero-point reset of this time, and changes the reset time interval to the next zero-point reset at regular intervals.

Preferably, the control device shortens the reset time interval with increase in difference of the output values.

Preferably, the control device elongates the reset time interval with decrease in difference of the output values.

Preferably, the control device changes the reset time interval according to the transition of the difference of the output values.

The weight measuring apparatus in the invention allows the reset time interval to the next zero-point reset to be changed according to the magnitude of the difference between the zero point set at the previous time and a newly obtained zero point. Therefore, when the change in the state of installation or the environment or the change with time occurs, the zero-point reset is carried out corresponding to the change in the state of installation or the like by shortening the reset time interval, so that accuracy of measurement of the weight is maintained at a high level. In contrast, when there is not much change in the state of installation or the like, power consumption of the weight measuring apparatus is significantly reduced by elongating the time interval to the next zero-point reset and reducing the number of times of the zero-point reset.

**In the Drawings;**
Fig. 1 is a perspective view of a body-fat-meter-integrated weighting machine according to an embodiment of the invention;
Fig. 2 is a block diagram of the body-fat-meter-integrated weighting machine in Fig. 1; and
Fig. 3 is a flowchart for explaining a process of zero-point reset for the body-fat-meter-integrated weighting machine in Fig. 1.

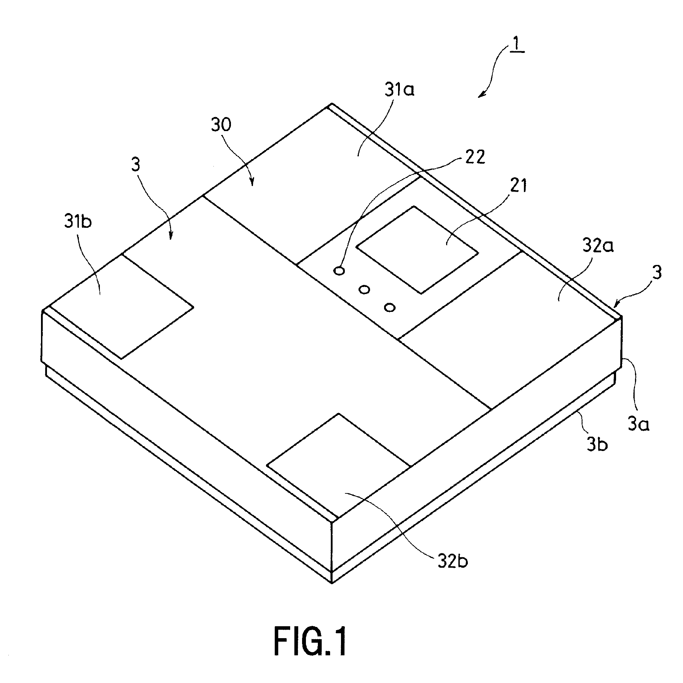

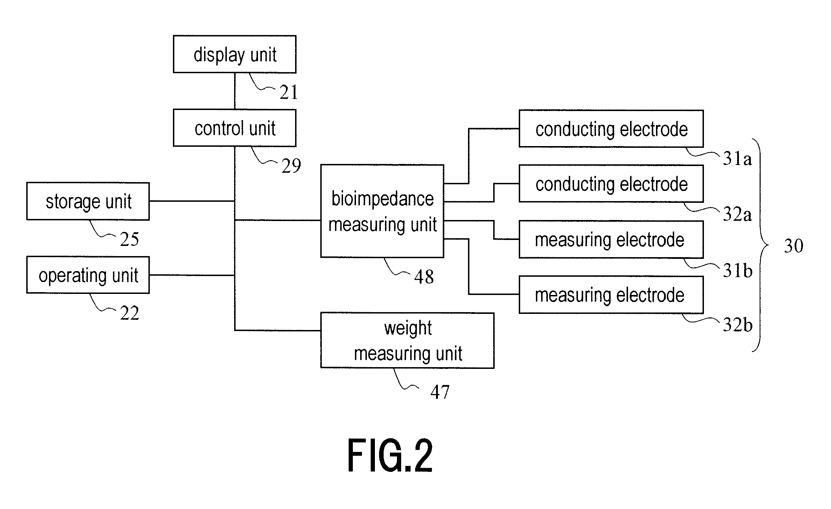

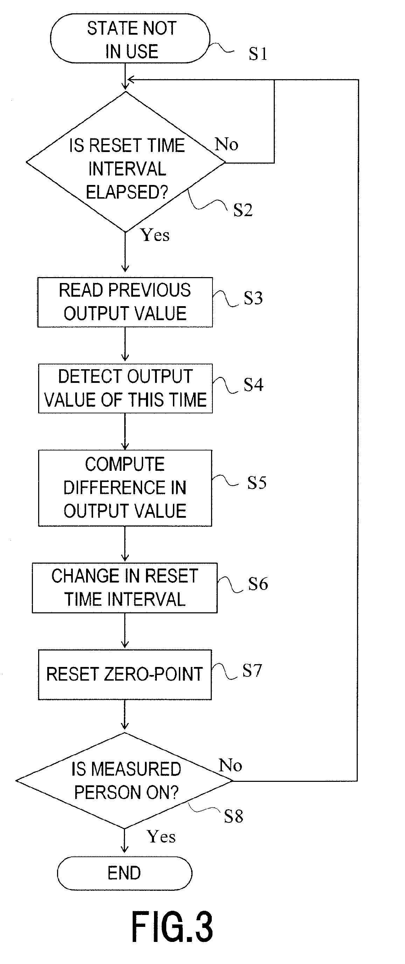

Referring now to the drawings, an embodiment in which the weight measuring apparatus according to the invention is applied to a body-fat-meter-integrated weighting machine of so-called step-on type. Fig. 1 is a perspective view showing a body-fat-meter-integrated weighting machine 1 according to the embodiment; Fig. 2 is a block diagram showing the body-fat-meter-integrated weighting machine in Fig. 1; Fig. 3 is a flowchart of a zero-point reset process for the body-fat-meter-integrated weighting machine in Fig. 1. A control unit (control device) 29, a storage unit 25, and a weight measuring unit 47 arranged in an interior of a machine body 3, being shown in Fig. 3, are omitted in Fig. 1 since they are internal mechanisms.

As shown in Fig. 1, the body-fat-meter-integrated weighting machine 1 (hereinafter, referred to as weighting machine 1) according to the embodiment includes the machine body 3 formed into a substantially box shape, and legs (not shown) provided on a back surface of the machine body 3 for supporting the machine body 3. The machine body 3 includes a bioimpedance measuring unit 48 having electrode members 30, the weight measuring unit 47, a display unit 21, an operating unit 22, the storage unit 25, the control unit 29, and a power source for supplying an electric power (not shown). Detailed configurations of the members are described in detail.

The machine body 3 is formed into a substantially box-shape by combining a cover member 3a and a bottom plate member 3b formed by molding resin (for example, ABS resin (acrylonitrile/butadiene/styrene copolymer)) or the like. Considering the strength of the weighting machine 1 as a product, the machine body 3 may be formed in combination of the cover member 3a formed of resin as described above and the bottom plate member 3b formed of metal.

As shown in Fig. 1, the thin plate-shaped four electrode members 30 (conducting electrodes 31a, 32a, measuring electrodes 31b, 32b) are held on an upper surface of the cover member 3a of the machine body 3, and are arranged apart from each other on the upper surface of the cover member 3a. Although a structure for holding the electrode members 30 may be selected as needed, for example, it is preferable to form recesses (not shown) for fitting the electrode members 30 on the cover member 3a, and hold the same in a state of being fitted so that the electrode members 30 and the cover member 3a are flush with each other (see Fig. 1).

As shown in Fig. 1, the cover member 3a of the machine body 3 includes the display unit 21 and the operating unit 22 in addition to the electrode members 30. The display unit 21 is a data display device for displaying data sent from the control unit 29, and displays mainly various biometric data of the measured person or operating guidance. As the display unit 21, for example, a liquid crystal display such as a full dot LCD (Liquid Crystal Display) is applicable. The operating unit 22 is an operating portion to be used for entering the biometric data of the measured person (for example, sex, age, or height) or various settings of the weighting machine 1. In the embodiment, the operating unit 22 having three buttons on the near side of the display unit 21 is employed as an example, the number and the shape of the operating member and the operating method are not specifically limited thereto, and may be selected from, for example, touch sensor type or dial type. A foot switch which allows the measured person to be operated with a foot may be provided on a side surface of the machine body 3. The display unit 21 and the operating unit 22 may be integrated as a liquid crystal display panel having a touch-panel function. The entered biometric data or set values of the measured person are stored in the storage unit 25 or are displayed on the display unit 21.

As shown in the block diagram in Fig. 2, the control unit 29 is electrically connected to the display unit 21, the operating unit 22, the weight measuring unit 47, and the bioimpedance measuring unit 48, and controls operations thereof. The control unit 29 is connected to a power source (not shown) and the storage unit (for example, Random Access Memory (RAM)) 25.

A battery or an external power source for supplying an electric power for activating the weighting machine 1 may be used as the power source. The weighting machine 1 in this embodiment is not supplied with the power required for a measuring process in a state in which the weighting machine 1 is not used for measurement (hereinafter referred to as a state not in use), and only the power required for the zero-point reset and determination of whether the measured person is on the weighting machine 1 or not, described later, is supplied. After having determined that the measured person is on the weighting machine 1, that is, in a state in which the weighting machine 1 is used for the measurement (hereinafter referred to as a state in use), the power required for the measurement is supplied. Therefore, in the state not in use, for example, no power is distributed to the display unit 21, and hence nothing is displayed.

The machine body 3 includes the weight measuring unit 47 (weight measuring unit) for measuring the load of the measured person on the machine body 3 (body weight, weight) provided in the interior thereof. More specifically, the weight measuring unit 47 may be a load cell which is made up, for example, of a distortable member formed of a metallic member which is distorted according to the applied load, and a strain gauge bonded to the distortable member. The machine body 3 is supported by one end of the distortable member, and the other end of the distortable member is supported by the leg portions (not shown). Accordingly, when the distortable member is distorted by the load applied when the measured person rides on the upper surface of the machine body 3, the strain gauge expands and contracts and value of resistance (output value) according to the expansion and contraction of the strain gauge changes, and the weight is measured on the basis of the change in resistance as a change in output of the load signal. In other words, the control unit 29 obtains the weight from the difference between the value of resistance (output value) (so-called zero-point) from the weight measuring unit 47 when no load is applied to the machine body 3, and the value of resistance (output value) when the load is applied thereto, so that the weight of the measured person is measured. The weight of the measured person obtained in this manner is stored in the storage unit 25 and is displayed on the display unit 21. As described later, the values of resistance (output value) from the weight measuring unit 47 when no load is applied to the machine body 3 (so-called zero point) are outputted to the control unit 29 at predetermined time intervals.

The bioimpedance measuring unit 48 includes a constant current feeding unit (not shown) connected to the conducting electrodes 31a and 32a which are adapted to come into contact with bottoms (toes) of the feet of the measured person for providing a predetermined weak constant current thereto and a voltage measuring unit (not shown) connected to the measuring electrodes 31b and 32b which are adapted to come into contact with the bottoms (heels) of the feet of the measured person for measuring potential difference of the living body. In this embodiment, the pair of conducting electrode 31a and the measuring electrode 31b are arranged so as to come into contact with the bottom of the left foot, and the pair of conducting electrode 32a and the measuring electrode 32b are arranged so as to come into contact with the bottom of the right foot. Accordingly, when the measured person rides on the weighting machine 1, the constant current feeding unit distributes a current from the conducting electrodes 31a and 32a between the toes via the both legs (lower body) and the potential difference (voltage) generated in this current path is measured between the heels, so that the bioimpedance of the measured person is obtained from a measured voltage value and an applied current value. The bioimpedance of the measured person obtained in this manner is outputted from the bioimpedance measuring unit 48 to the control unit 29.

After having determined the fact that the measured person rides on the machine body 3 of the weighting machine 1, the control unit 29 immediately gives an instruction to start measurement of the weight by the weight measuring unit 47 and measurement of the bioimpedance by the bioimpedance measuring unit 48, respectively. Although means for determining the fact that the measured person rides on the machine body 3 of the weighting machine 1 is selectable as needed, a method of monitoring the change in output value from the weight measuring unit 47 in the state not in use by the control unit 29, and determining the fact that the measured person rides on the weighting machine 1 at a moment when a sudden change in the output value appears, or at a moment when the output value exceeds the predetermined value may be employed for example.

The control unit 29 computes by applying parameters such as the weight obtained by the weight measuring unit 47, the bioimpedance obtained by the bioimpedance measuring unit 48 and the biometric information of the measured person entered via the operating unit 22 (for example, sex, age and height) to a regression formula predetermined for calculating the percent body fat and other biometric data. The biometric data obtained by such computation includes various types of values such as the offal fat level, the body water volume, the muscle mass, the basal metabolic rate, the bone mass, the fat-free mass, the body cell mass, the blood pressure, the visceral fat area, BMI (Body Mass Index), the degree of obesity, the intracellular fluid volume, and the extracellular fluid in addition to the percent body fat, which may be selected as needed according to the object of usage of the weight measuring apparatus according to the invention. The biometric information such as the percent body fat obtained by computation is displayed on the display unit 21 or stored in the storage unit 25.

The control unit 29 carries out the so-called zero-point reset when the weighting machine 1 is not in use. The control unit 29 gives an instruction to the weight measuring unit 47 to output the output values in the unloaded state of the machine body 3 the reset time intervals, and stores the same to the storage unit 25.

Referring now to Fig. 3, the process of the zero-point reset of the weighting machine 1 will be described below. The weighting machine 1 may be adapted to be able to set initial values of the zero point and the reset time intervals to suitable values in advance when shipping a product, and may be adapted to be reset by the measured person when using the weighting machine 1 for the first time.

First of all, with reference to the state of the weighting machine 1 not in use (Step S1), a timer in the control unit 29 counts the time elapsed from the moment when the weighting machine 1 is brought into the state not in use, and the control unit 29 determines whether or not the currently set reset time interval is elapsed (Step S2). When the reset time interval is elapsed, the control unit 29 reads an output signal value which indicates the current zero point stored in the storage unit 25 (output value of the load signal at the time of previous zero-point reset) (Step S3), and detects a load signal output value (output value of the load signal at the time of zero-point reset of this time) from the load cell as the weight measuring unit 47 used at the zero-point reset of this time (Step S4).

Subsequently, the control unit 29 computes the difference between the load signal output value which indicates the read current zero point and the newly detected load signal output value (Step S5). The currently set reset time interval is changed to a new reset time interval according to the difference of the output values calculated by the image data area 29 (Step S6), and a newly detected load signal output value is stored in the storage unit 25 as latest zero-point data, so that the zero-point reset is achieved (Step S7). In this embodiment, the difference in the output value of the load signal from the load cell as the weight measuring unit 47 is equal to or larger than the predetermined value, the control unit 29 sets the time period until the next zero-point reset (reset time period) to a value shorter than the previous reset time period. In this manner, the fact that the difference of the load signal outputs is rather large is considered to be because the weighting machine 1 is in a state in which the zero point is changed due to the change in the state of installation (posture) (for example, the case in which the posture is changed from a state of being propped against a wall or the like to a state of being placed horizontally on the floor, or inverted when a battery as a power source is replaced), the change in the environment such as the peripheral temperature of a place where the weighting machine 1 is arranged (for example, minute difference of distortion depending on temperature characteristics of the distortable member used for the load cell), the change with time (for example, minute displacement of a fixed state of the load cell with respect to the machine body 3 due to the impact or the like) and hence the difference of the output values of the load cell is resulted. Therefore, the configuration in which the reset time interval is shortened so as to be able to measure the biometric data (weight, in particular) using the latest zero point corresponding to the changes as such is employed so that the zero-point reset is carried out often.

In contrast, when the difference of the load signal output values is smaller than the predetermined value, the control unit 29 determines that there is no specific change in the state of installation or the environment of the weighting machine 1, and changes the reset time interval to the next zero-point reset to be longer than the previous reset time interval (Step S6), and resets the load signal output value obtained at this time (the output value of the load signal at the time of zero-point reset at this time) as a new zero point (Step S7). The fact that the difference of the load signal output value is rather small is considered to be because the state of installation or the environment of the weighting machine 1 are not changed. Therefore, the zero-point reset does not have to be carried out very often, and saving of the power consumed by the zero-point reset is advantageously achieved by setting the reset time interval to a long interval.

The reset time interval to be set newly may be elongated or shortened to different values every time when the zero-point reset is carried out in such a manner that (1) the reset time interval is elongated or shortened always by a constant interval when the fact that the difference is generated in the load signal output value is detected or (2) the reset time interval is reduced as the difference in the output value increases and is elongated as the difference in the output value decreases, that is, the more the difference in the output value is, the shorter the value of the reset time interval to be set becomes, and the smaller the difference in the output value is, the longer the value of the reset time interval to be set becomes. Alternatively, it is also possible that (3) the difference in the output value to be computed at the time of every zero-point reset is stored in the storage unit 25 as data and the reset time interval is changed according to the transition thereof. In this case, for example, when the difference in the output value computed at the latest several times of zero-point reset is gradually increased, the reset time interval is set to a short value, and when it is gradually decreased, the reset time interval is set to a long value. When the case (1) is employed, the processing in the control unit 29 is simplified, and the programming is easily achieved, and hence the production cost is reduced. When the cases (2) and (3) are employed, further adequate accommodation is advantageously achieved when the change in the state of installation or the environment or the change with time occurs or they are stabled.

Finally, the determination whether the measured person rides on the machine body 3 or not is carried out in the control unit 29 (Step S8). When it is determined that the measured person is on the machine body 3 (Yes in Step S8), the zero-point reset is ended, and the weighting machine 1 is brought into the state in use. In contrast, when the control unit 29 determines that the measured person is not on the machine body 3 (No in Step S8), the procedure goes back to Step S2 described above, where the zero-point reset process (Step S2 to Step S7) is repeated.

As described above, the weighting machine 1 in the embodiment has the configuration to change the reset time interval for the zero-point reset according to the presence or absence of the change in the state of installation or the environment of the weighting machine 1 or the change of the weighting machine 1 by itself with time, so that the biometric information is measured by resetting the zero point accurately with high degree of accuracy and, simultaneously, the power consumption is restrained.

A configuration in which when the difference between the load signal output value obtained at the time of previous zero-point reset and the load signal output value obtained at the zero-point reset of this time is equal to or larger than the predetermined value, the message which notifies abnormality at the weight measuring unit to the measured person on the display unit is also applicable.

The change of the zero point of the load cell as the weight measuring unit depends on the temperature characteristic of the load cell (specifically, the distortable member) in many cases. Therefore, a configuration in which a temperature sensor is provided in the weighting machine in the embodiment is also applicable. In this case, a configuration in which the relation between the temperature and the load signal output value of the load cell (temperature characteristic) is acquired in advance, and the reset time interval to the next time is changed and set on the basis of the difference between the load signal output value acquired at the time of zero-point reset which is an output value after having corrected the error due to the temperature difference and the load signal output value indicating the zero point which is reset at the time of the previous zero-point reset is also applicable. In this configuration, the influence of the temperature change, which is a main cause of the change of the zero point, is eliminated, so that the frequency to shorten the reset time interval for resetting the zero point may be reduced, and hence further reduction of the amount of consumption of a waiting power is achieved.

In the embodiment shown above, the case in which the weight measuring apparatus in the invention is applied to the body-fat-meter-integrated weighting machine has been described. However, the invention is also applicable to the weighting machine only for weighing the weight and, in contrast, the invention is also applicable to the body-composition-meter-integrated weighting machine which is able to obtain various biometric data including the body fat. The weight measuring apparatus in the invention is applicable not only to the biometric apparatus aimed at living bodies as the object of measurement, such as the weighting machine shown in the embodiment described above, but also to the apparatus for measuring or weighing the weight of substances such as a mass meter (such as a kitchen scale). The weight measuring unit applicable in this case is a load cell including the distortable member and the strain gauge bonded to the distortable member as in the case of the weight measuring unit 47 in the weighting machine 1 in the embodiment shown above.

The invention may be embodied in various modes without departing its essential features. Therefore, needless to say, the embodiment shown above is illustrative only, and does not limit the invention.

## Claims

1. A weight measuring apparatus which carries out zero-point reset as an output value of a load signal in an unloaded state when the weight measuring apparatus is not in use, comprising:
a weight measuring unit that measures a load applied to a machine body and outputs the load signals; and
a control device that receives the load signals from the weight measuring unit at reset time intervals for carrying out the zero-point reset of the weight measuring unit in the unloaded state,
wherein the control device changes the reset time interval to next zero-point reset according to the difference between the output value of the load signal at the time of previous zero-point reset and the output value of the load signal at zero-point reset of this time.

2. The weight measuring apparatus according to Claim 1, wherein the control device elongates the reset time intervals when the difference of the output values is smaller than a predetermined value, and shortens the reset time interval when the difference of the output values is equal to or larger than the predetermined value.

3. The weight measuring apparatus according to Claim 1 or Claim 2, wherein the control device detects the difference between the output value of the load signal at the previous zero-point reset and the output value of the load signal at the zero-point reset of this time, and changes the reset time interval to the next zero-point reset at regular intervals.

4. The weight measuring apparatus according to Claim 1, wherein the control device shortens the reset time interval with increase in difference of the output values.

5. The weight measuring apparatus according to Claim 1 or Claim 4, wherein the control device elongates the reset time interval with decrease in difference of the output values.

6. The weight measuring apparatus according to Claim 1, wherein the control device changes the reset time interval according to the transition of the difference of the output values.
